# EUROPEAN PATENT APPLICATION

(11) **EP 3 118 809 A1**
(43) Date of publication of application: **18.01.2017**
(21) Application number: 15762106.1
(22) Date of filing: 10.03.2015
(51) Int. Cl.: G06Q 50/22

(54) **TEAM MEDICAL SUPPORT DEVICE, CONTROL METHOD FOR TEAM MEDICAL SUPPORT DEVICE, TEAM MEDICAL SUPPORT PROGRAM, AND TEAM MEDICAL SUPPORT SYSTEM**

(30) Priority: 13.03.2014 JP 2014050346
(71) Applicant: Fujifilm Corporation, Minato-ku Tokyo 106-8620 (JP)
(72) Inventor: KUDOU,Yuuya, Tokyo 107-0052 (JP); UEDA, Satoshi, Tokyo 107-0052 (JP); MATSUMASA, Hironori, Tokyo 107-0052 (JP); OHTA, Yasunori, Tokyo 107-0052 (JP)
(74) Representative: Klunker . Schmitt-Nilson . Hirsch
(86) International application number: PCT/JP2015/057038
(87) International publication number: WO 2015/137350

(57) **Abstract**

Provided are a team medical support device, a method for controlling a team medical support device, a team medical support device control program, and a team medical support system which can smoothly perform information exchange and information sharing in a team medical treatment.

A team medical support device (11) reads patient schedule information and a communication message from a patient schedule information storage unit (16) and a communication message storage unit (18) in response to a transmission request from client terminals (13a) to (13c), creates a medical support screen (20), and transmits the medical support screen (20) to the client terminals (13a) to (13c). A patient schedule information display region (20a) and a communication message display region (20b) in which communication messages related to a patient or event information for the patient are summarized is displayed on the medical support screen (20).

## Description

### TECHNICAL FIELD

The present invention relates to a team medical support device, a method for controlling a team medical support device, a team medical support program, and a team medical support system which support a team medical treatment in which a plurality of medical staff members examine a patient in cooperation with each other.

In the medical field, a team medical treatment in which a medical team including medical staff members, such as doctors or nurses, examines a patient is performed. In addition, the use of a system in which patient schedule information (which is also referred to as a clinical path) indicating a plan for examining a patient is introduced into a team medical treatment and a medical team examines a patient on the basis of the patient schedule information has increased. The plan for examining a patient is a plan for a medical procedure that is scheduled to be performed for a patient, such as an examination, a cure, or a medical treatment. Event information indicating a medical procedure, such as an examination, a cure, or a medical treatment, is recorded in the patient schedule information in chronological order. In the team medical treatment, it is important to smoothly exchange and share information between a plurality of medical staff members and thus to strengthen the cooperation between the medical staff members. As a device that can be used for smooth information exchange and sharing between medical staff members, JP2007-249251A (US2008/021738A) discloses a clinical communication device.

In the clinical communication device disclosed in JP2007-249251A (US2008/021738A), patient schedule information and staff schedule information, which is a medical staff work schedule, are managed in a database. The staff schedule information is schedule information indicating the relationship between the patient and work that the medical staff is in charge of. Event information indicating the work performed by the medical staff is recorded in the staff schedule information in chronological order.

The clinical communication device can be accessed by a client terminal of the medical staff. In case the client terminal accesses the clinical communication device, a medical staff member designates his or her staff ID and transmits a transmission request including designation information to the clinical communication device. For the staff ID, for example, in a case in which the client terminal is allocated to each medical staff member and the terminal ID of the client terminal corresponds to the staff ID, the terminal ID is given as staff ID designation information to the transmission request. The clinical communication device creates a communicator screen in which the patient schedule information and the staff schedule information are combined on one screen and transmits the communicator screen, in response to a request from the client terminal. On the communicator screen, a staff schedule display region in which the staff schedule information of a designated medical staff member is displayed and a patient schedule information display region in which the patient schedule information of a plurality of patients that the designated medical staff member is in charge of is displayed are provided in parallel in one screen. The schedules of a plurality of patients are displayed side by side in the patient schedule information display region.

In the communicator screen disclosed in JP2007-249251A (US2008/021738A), in a case in which there is a communication message related to a certain patient, such as an item to be transmitted between medical staff members, a message mark indicating whether a communication message is present is displayed in the patient schedule information display region so as to be associated with the patient schedule information. In case the message mark is clicked, the text of the communication message is displayed.

According to the clinical communication device, a medical staff member can check, for example, the patient schedule information of the patient that the medical staff member is in charge of, in addition to his or her staff schedule information, through the client terminal. Furthermore, a medical staff member can exchange a communication message with other medical staff members through the communicator screen. In a team medical treatment, since a plurality of medical staff members are related to one patient, each medical staff member can check the patient schedule information and exchanges communication messages. Therefore, the medical staff members smoothly exchange and share information and it is possible to strengthen the cooperation between a plurality of medical staff members.

### SUMMARY OF THE INVENTION

However, the techniques disclosed in JP2007-249251A (US2008/021738) is not enough to achieve smooth information exchange and information sharing in the team medical treatment. First, in the clinical communication device disclosed in JP2007-249251A (US2008/021738), a plurality of patient schedule information items and staff schedule information items are displayed in parallel on one screen and a message mark to be addressed to each medical staff member is also displayed for each patient schedule information item in the patient schedule information display region. Therefore, the type or number of information items is too large and the information is difficult to see.

Second, in the clinical communication device disclosed in JP2007-249251A (US2008/021738), it is difficult to collectively check the communication messages related to a specific patient or a specific medical procedure. In the team medical treatment, there are the following cases: a case in which the communication messages related to the entire medical examination for a patient are exchanged; and a case in which a medical procedure that is scheduled to be performed for a patient is specified and communication messages are exchanged. Therefore, in a case in which the entire plan for examining a patient is checked and reviewed, there is a demand for collectively checking the communication messages related to the patient. In a case in which it is necessary to rapidly and accurately ascertain the content of the medical procedure that is scheduled to be performed for a patient, there is a demand for collectively checking only the communication messages related to the medical procedure.

However, in the clinical communication device disclosed in JP2007-249251A (US2008/021738), in case the message mark is clicked, only one communication message which has been transmitted from a specific medical staff member to another medical staff member is displayed and it is difficult to understand the relation between a plurality of communication messages. Therefore, in the clinical communication device disclosed in JP2007-249251A (US2008/021738), in order to check a plurality of communication messages while ascertaining the relation between the communication messages and a specific patient or a specific medical procedure, it is necessary to click a plurality of message marks to display the content of the communication messages, to read, for example, the transmission date and time, transmission source, transmission destination, title, and text of each of the displayed communication messages, and to check the content of the communication messages, which is very complicated.

In the team medical treatment, it is important to rapidly and accurately ascertain the relation between a plurality of communication messages as well as the content of one communication message. However, as described above, in the clinical communication device disclosed in JP2007-249251A (US2008/021738), the following problems need to be solved: since the type or number of information items displayed on one screen is large, the information is difficult to see; and it is difficult to collectively check the communication messages related to a specific patient or a specific medical procedure.

An object of the invention is to provide a team medical support device, a method for controlling a team medical support device, a team medical support device control program, and a team medical support system which can smoothly perform information exchange and information sharing in a team medical treatment.

In order to solve the above-mentioned problems, a team medical support device according to the invention comprises a patient schedule information storage unit, a communication message storage unit, a receiving unit, a screen creation unit, and a transmission unit. The patient schedule information storage unit stores patient schedule information of a plurality of patients in which event information indicating a medical procedure that is performed for the patient is registered. The communication message storage unit stores a plurality of communication messages which are exchanged between client terminals used by a plurality of medical staff members. The receiving unit receives a transmission request including patient designation information for designating one patient among the plurality of patients. The screen creation unit creates a medical support screen comprising a patient schedule information display region in which the patient schedule information of the patient designated by the patient designation information among the patient schedule information items of the plurality of patients is displayed and a communication message display region in which a communication message related to the patient designated by the patient designation information or a communication message related to at least one event information item of the patient designated by the patient designation information among the plurality of communication messages is summarized and displayed. The transmission unit transmits the medical support screen to the client terminal which has transmitted the transmission request.

Preferably, in a case in which the screen creation unit creates the medical support screen including the communication message display region in which the communication message related to at least one event information item is summarized, the screen creation unit creates a medical support screen on which only the patient schedule information display region is displayed in response to a transmission request. Preferably, in a case in which at least one event information item is selected from the medical support screen transmitted to the client terminal, the screen creation unit re-creates the medical support screen comprising the communication message display region in which a communication message related to the selected event information item is summarized and displayed.

It is preferable that the communication message display region is displayed so as to be superimposed on the patient schedule information display region and is connected to the event information selected in the patient schedule information display region by a leader line.

It is preferable that the communication message display region is displayed in parallel to the patient schedule information display region.

It is preferable that a transmission date and time of the communication message is displayed in the communication message display region. A transmission source and a transmission destination of the communication message may be displayed in the communication message display region. A title of the communication message or at least a portion of text of the communication message may be displayed in the communication message display region. It is preferable that the communication messages are displayed in the communication message display region in an order of transmission date and time.

Preferably, the team medical support device further comprises an access authority information storage unit that stores access authority information of the medical staff member for the communication message display region. Preferably, in case of creating the medical support screen, the screen creation unit checks the access authority information of the medical staff member who is a request source of the transmission request. Preferably, in a case in which the communication messages displayed in the communication message display region include a communication message that is not allowed to be accessed by the medical staff member, the screen creation unit performs a concealment process of concealing the communication message that is not allowed to be accessed. It is preferable that the concealment process includes a process of displaying information indicating that the communication message which is not allowed to be accessed by the medical staff member is present.

Preferably, the team medical support device further comprises a message relay unit that receives the communication message transmitted from the client terminal and relays the communication message and a reception notification unit that transmits a notification indicating the reception of the communication message to the client terminal used by the medical staff member who is a transmission destination of the received communication message.

A method for controlling a team medical support device according to the invention comprises a receiving step, an information reading step, a screen creation step, and a transmission step. In the receiving step, a transmission request including patient designation information is received. In the information reading step, a patient schedule information storage unit and a communication message storage unit are accessed and the patient schedule information of the patient designated by the transmission request and the communication message are read. In the screen creation step, a medical support screen comprising a patient schedule information display region in which the patient schedule information read in the information reading step is displayed and a communication message display region in which a communication message related to the patient designated by the patient designation information or a communication message related to at least one event information item of the patient designated by the patient designation information among the communication messages read in the reading step is summarized and displayed is created. In the transmission step, the medical support screen is transmitted to the client terminal which has transmitted the transmission request.

A method for controlling a team medical support device according to the invention causes a computer to perform a receiving step, an information reading step, a screen creation step, and a transmission step.

A team medical support system according to the invention comprises client terminals that are used by a plurality of medical staff members and a team medical support device that creates a medical support screen on the basis of a transmission request from the client terminals and transmits the medical support screen to the client terminals. Of these, the team medical support device comprises a patient schedule information storage unit, a communication message storage unit, a receiving unit, a screen creation unit, and a transmission unit.

### EFFECT OF THE INVENTION

The team medical support device according to the invention creates the medical support screen comprising the patient schedule information display region in which the patient schedule information of the patient designated by the transmission request is displayed and the communication message display region in which the communication message related to the patient designated by the patient designation information or the communication message related to at least one event information item of the patient designated by the patient designation information is summarized and displayed, in response to the transmission request from the client terminals used by the medical staff members, and transmits the medical support screen to the client terminals which have transmitted the transmission request. Only the patient schedule information of one patient and the communication message related to a designated patient or at least one event information item are displayed on the medical support screen. Therefore, the content is concisely displayed and it is easy to ascertain the relation between the patient schedule information and the communication message. In addition, in a case in which the communication messages related to a patient are summarized and displayed, it is possible to collectively check the communication messages related to the entire plan for examining a patient and it is easy to check or review the entire plan for examining a patient. In a case in which the communication messages related to event information are summarized and displayed, it is possible to collectively check the communication messages related to a medical procedure for a patient which is indicated by the event information. Therefore, it is possible to accurately and rapidly ascertain the content of the communication messages related to the medical procedure. As a result, the medical staff members smoothly exchange and share information in a team medical treatment.

### BRIEF DESCRIPTION OF THE DRAWINGS

Fig. 1 is a diagram illustrating the structure of a team medical support system according to a first embodiment.
Fig. 2 is a diagram illustrating a table of a patient schedule information storage unit.
Fig. 3 is a diagram illustrating a table of a communication message storage unit.
Fig. 4 is a diagram illustrating a table of a patient information storage unit.
Fig. 5 is a diagram illustrating a table of a staff information storage unit.
Fig. 6 is a block diagram schematically illustrating the electrical structure of a computer forming the team medical support device.
Fig. 7 is a functional block diagram illustrating the computer in a case in which a team medical support program starts.
Fig. 8 is a diagram illustrating an operation menu screen.
Fig. 9 is a diagram illustrating a transmission request screen.
Fig. 10 is a diagram illustrating a medical support screen comprising only a patient schedule information display region.
Fig. 11 is a diagram illustrating a medical support screen comprising a communication message display region.
Fig. 12 is a diagram illustrating a message transmission screen.
Fig. 13 is a flowchart illustrating a medical support screen transmission process.
Fig. 14 is a diagram illustrating a medical support screen according to a second embodiment.
Fig. 15 is a diagram illustrating a state in which the text of a communication message is displayed on the medical support screen according to the second embodiment.
Fig. 16 is a diagram illustrating a state in which a communication message related to event information is displayed on the medical support screen according to the second embodiment.
Fig. 17 is a diagram illustrating the structure of a team medical support system according to a third embodiment.
Fig. 18 is a flowchart illustrating a medical support screen transmission process according to the third embodiment.
Fig. 19 is a diagram illustrating a medical support screen according to the third embodiment.
Fig. 20 is a diagram illustrating the structure of a team medical support system according to a fourth embodiment.
Fig. 21 is a flowchart illustrating a communication message reception process according to the fourth embodiment.

### DESCRIPTION OF THE PREFERRED EMBODIMENTS

### [First Embodiment]

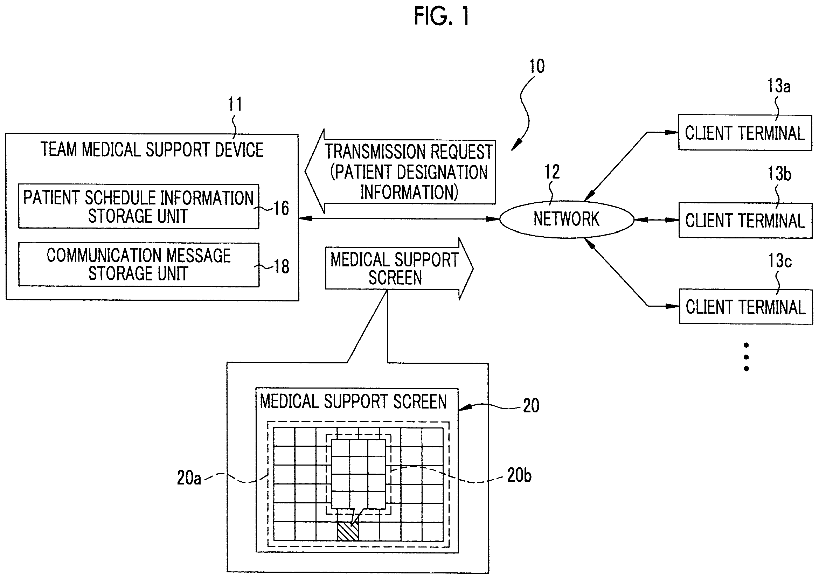

A team medical support system 10 illustrated in Fig. 1 is a client-server computer system that supports a team medical treatment in which a medical team including a plurality of medical staff members, such as doctors or nurses, examines patients and enables the medical staff members to smoothly exchange and share information therebetween. The team medical support system 10 comprises a team medical support device 11 that is installed in, for example, an information processing room of a medical institution, such as a hospital, and a plurality of client terminals 13a to 13c that are connected to the team medical support device 11 through a network 12. The client terminals 13a to 13c are desktop or notebook personal computers or portable terminals, such as mobile phones, smart phones, or tablet terminals, and are used by the medical staff in the medical institution. The network 12 is a local area network (LAN) that is constructed in the medical institution.

The team medical support device 11 comprises a patient schedule information storage unit 16 and a communication message storage unit 18. The patient schedule information storage unit 16 stores patient schedule information of a plurality of patients indicating a patient examination plan which is generally called a clinical path. The patient examination plan is a plan for a medical procedure, such as an examination, a cure, or a medical treatment, which is scheduled to be performed for a patient. Event information indicating a medical procedure is registered in the patient schedule information. The communication message storage unit 18 stores a plurality of communication messages, such as messages related to a patient, which are exchanged between the medical staff members through the client terminals 13a to 13c.

In a case in which a request to transmit a medical support screen 20 is received from the client terminals 13a to 13c, the team medical support device 11 creates the medical support screen 20 on the basis of the patient schedule information of a patient designated by patient designation information included in the transmission request and a communication message. The medical support screen 20 is used to support medical staff members such that the medical staff members smoothly exchange and share information and comprises a patient schedule information display region 20a and a communication message display region 20b. The patient schedule information of the patient designated by the transmission request is displayed in the patient schedule information display region 20a in the form of a table in which patient event information is arranged in chronological order. In this embodiment, communication messages which are related to event information selected from patient schedule information items displayed in the patient schedule information display region 20a are summarized and displayed in the communication message display region 20b.

The team medical support device 11 transmits the medical support screen 20 created in response to the transmission request to the client terminals 13a to 13c which have transmitted the transmission request. The medical support screen 20 transmitted to the client terminals 13a to 13c is displayed on displays of the client terminals 13a to 13c. Therefore, the medical staff members can refer to the medical support screens 20 of the client terminals 13a to 13c and can smoothly exchange and share information required for a team medical treatment.

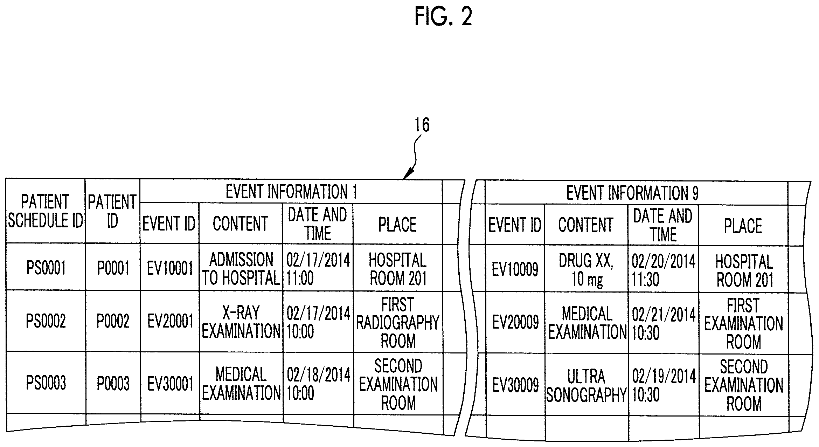

As illustrated in Fig. 2, the patient schedule information storage unit 16 is, for example, a table-type database in which the patient schedule information of each patient is stored in each record. A patient schedule ID, a patient ID, and a plurality of event information items are stored in each record of the patient schedule information storage unit 16. The patient schedule ID is an ID for identifying patient schedule information and includes letters "PS" and an identification number. The patient ID is an ID for identifying a patient and includes a letter "P" and an identification number. The event information includes an event ID for identifying each event information item, the content of a medical procedure, a date and time, and a place. The event ID includes letters "EV" and an identification number.

For example, the patient schedule information of a patient with a patient ID "P0001" is stored in a record corresponding to a patient schedule ID "PS0001" and an event ID "EV10001", content "admission to hospital", a date and time "2014/02/17 10: 00", and a place "hospital room 201" are registered in event information 1. In addition, an event ID "EV10009", content "drug XX, 10 mg" , a date and time "2014/02/20 11:30", a place "hospital room 201" are registered in event information 9 of the same patient. The patient schedule information of a patient with a patient ID "P0003" is stored in a record corresponding to a patient schedule ID "PS0003" and an event ID "EV30001" , content "X-ray examination" , a date and time "2014/02/17 10: 00" , and a place "first radiography room" are registered in event information 1.

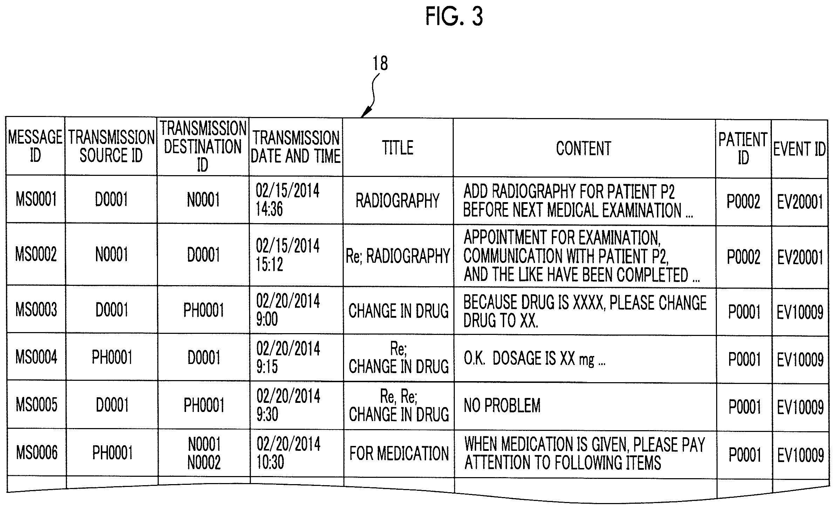

As illustrated in Fig. 3, the communication message storage unit 18 is, for example, a table-type database in which each communication message exchanged among the client terminals 13a to 13c is stored in each record. A message ID, a transmission source ID, a transmission destination ID, a transmission date and time, a title, text, a patient ID, and an event ID are registered in each record of the communication message storage unit 18.

The message ID is an ID for identifying a communication message and includes letters "MS" and an identification number. The transmission source ID and the transmission destination ID are a staff ID of a medical staff member who has transmitted a communication message and a staff ID of a medical staff member who is the destination of the communication message, respectively. The staff ID includes letters indicating the job type of a medical staff member and an identification number. For the letters indicating the job type of the medical staff, for example, "D" indicates a doctor, "N" indicates a nurse, "E" indicates an X-ray or computed tomography (CT) technician, and "PH" indicates a pharmacist. The patient ID is an ID of the patient related to a communication message. For the event ID, an event ID of event information related to a communication message is registered.

For example, a transmission source ID "D0001", a transmission destination ID "PH0001", a transmission date and time "2014/02/20 9: 00", a title "change in drug", a text "Please, change drug from XXXX to XX.", a patient ID "P0001", and an event ID "EV10009" are registered in a record corresponding to a message ID "MS0003". In addition, a reply to a communication message with the message ID "MS0003" is stored in a record corresponding to a message ID "MS0004" and a reply to a communication message with the message ID "MS0004" is stored in a record corresponding to a message ID "MS0005". A communication message which corresponds to the communication messages with the message IDs "MS0003" to "MS0005" and has been newly transmitted from the transmission source ID "PH0001" to transmission destination IDs "N0001" and "N0002" is stored in a record corresponding to a message ID "MS0006". Since the communication messages with the message IDs "MS0003" to "MS0006" are related to the same event information, the same event ID "EV10009" is recorded for the communication messages.

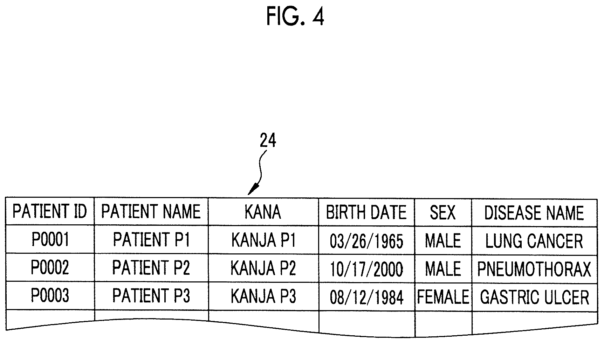

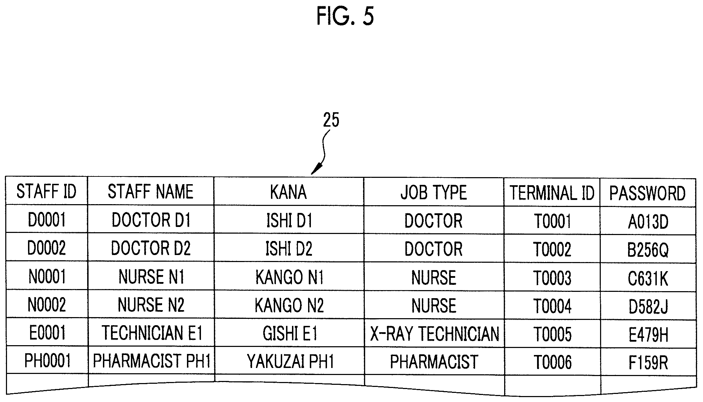

As illustrated in Figs. 4 and 5, the team medical support device 11 comprises a patient information storage unit 24 that stores the personal information of patients and a staff information storage unit 25 that stores the personal information of the medical staff, in addition to the patient schedule information storage unit 16 and the communication message storage unit 18. In case of creating the medical support screen 20, the team medical support device 11 reads information which is related to patients or the medical staff and has not been stored in the patient schedule information storage unit 16 and the communication message storage unit 18 from the patient information storage unit 24 and the staff information storage unit 25.

The patient information storage unit 24 is a table-type database in which the personal information of patients is registered in each record. For example, a patient ID, a patient name, the Kana of the patient name, a birth date, sex, and a disease name are registered in each record of the patient information storage unit 24.

For example, a patient name "patient P1", Kana "Kanja P1", a birth date "1965/03/26", sex "male", and a disease name "lung cancer" are registered in a record corresponding to a patient ID "P0001". In addition, a patient name "patient P3", Kana "Kanja P3", a birth date "1984/08/12", sex "female", and a disease name "gastric ulcer" are registered in a record corresponding to a patient ID "P0003".

The staff information storage unit 25 is a table-type database in which the personal information of each medical staff member is registered in each record. For example, a staff ID, a staff name, the Kana of the staff name, a job type, such as a doctor or a nurse, a terminal ID for identifying a client terminal that is used, and a password which is used to log in the team medical support device 11 are stored in each record of the staff information storage unit 25.

For example, a staff name "doctor D1", Kana "Ishi D1", a job type "doctor", a terminal ID "T0001", and a password "A013D" are registered in a record corresponding to a staff ID "D0001". In addition, for example, a staff name "nurse N1", Kana "Kango N1", a job type "nurse", a terminal ID "T0003", and a password "C631K" are registered in a record corresponding to a staff ID "N0001". Similarly, the staff information of an X-ray technician and the staff information of a pharmacist are stored in a record corresponding to a staff ID "E0001" and a record corresponding to a staff ID "PH0001", respectively.

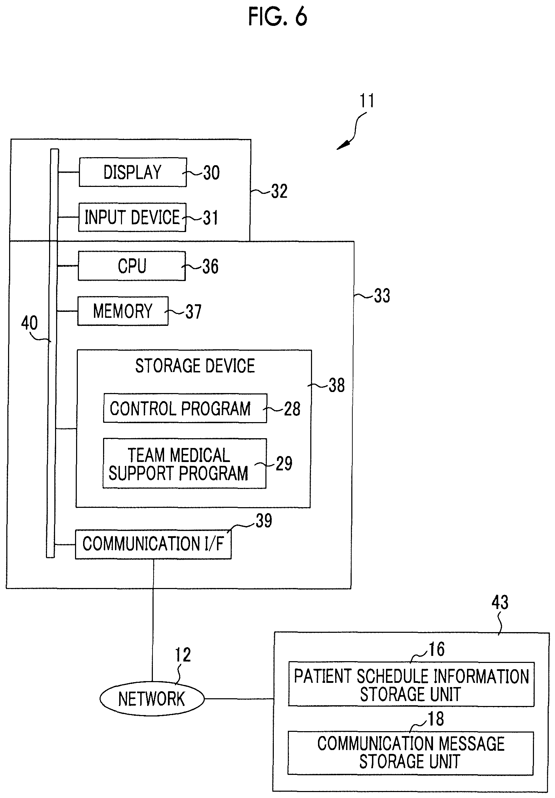

As illustrated in Fig. 6, the team medical support device 11 is configured by installing a control program 28, such as an operating system, or a team medical support program 29, which is an application program that causes a computer to function as the team medical support device 11, in a computer, such as a personal computer or a workstation. The team medical support device 11 comprises a console 32 including a display 30 and an input device 31, such as a keyboard, a mouse, or a touch panel, and a main body 33.

The main body 33 of the team medical support device 11 includes a CPU 36, a memory 37, a storage device 38, and a communication I/F 39 which are connected to each other through a data bus 40.

The storage device 38 is a device that stores various kinds of data and is, for example, a hard disk drive. The storage device 38 stores various kinds of programs, such as the control program 28 and the team medical support program 29.

The memory 37 is a work memory that is used by the CPU 36 to perform a process. The CPU 36 loads the control program 28 stored in the storage device 38 to the memory 37 and performs a process according to the program to control the overall operation of each unit of the computer. The communication I/F 39 is a communication interface that is connected to the network 12 and is used to communicate with the client terminals 13a to 13c. In addition, the communication I/F 39 is connected to a data server 43 in which the patient schedule information storage unit 16 and the communication message storage unit 18 are constructed through the network 12 such that it can communicate with the data server 43. The data server 43 is an external storage device. Of course, the patient schedule information storage unit 16 and the communication message storage unit 18 may be constructed in the storage device 38.

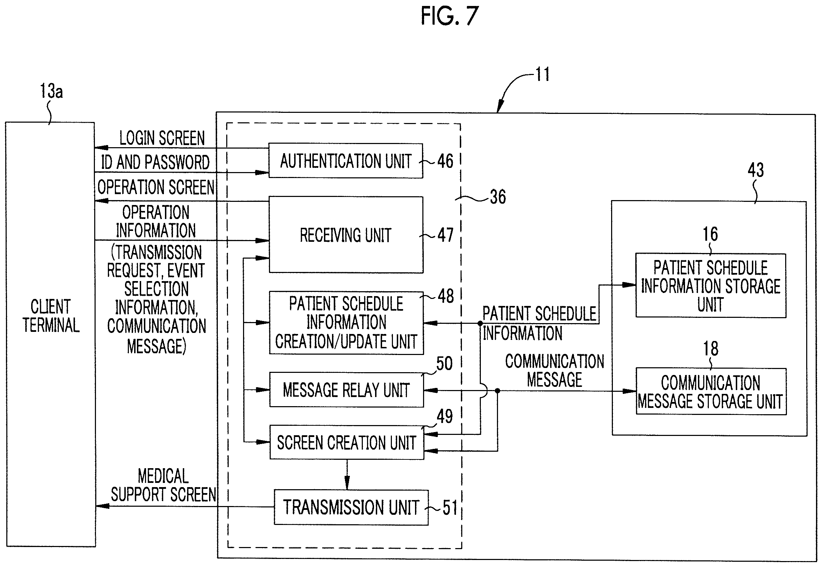

As illustrated in Fig. 7, in case the team medical support program 29 starts, the CPU 36 of the team medical support device 11 functions as an authentication unit 46, a receiving unit 47, a patient schedule information creation/update unit 48, a screen creation unit 49, a message relay unit 50, and a transmission unit 51 in cooperation with, for example, the memory 37.

The authentication unit 46 performs a process of authenticating the client terminals 13a to 13c that have accessed the team medical support device 11. In the authentication process, the authentication unit 46 transmits a login screen that prompts the input of a user ID and a password for login to the client terminals 13a to 13c. The authentication unit 46 compares the user ID and the password input to the login screen with the staff ID and the password stored in the staff information storage unit 25, allows the access of the client terminals 13a to 13c in a case in which the user ID and the password are identical to the staff ID and the password, and does not allow the access in a case in which the user ID and the password are not identical to the staff ID and the password.

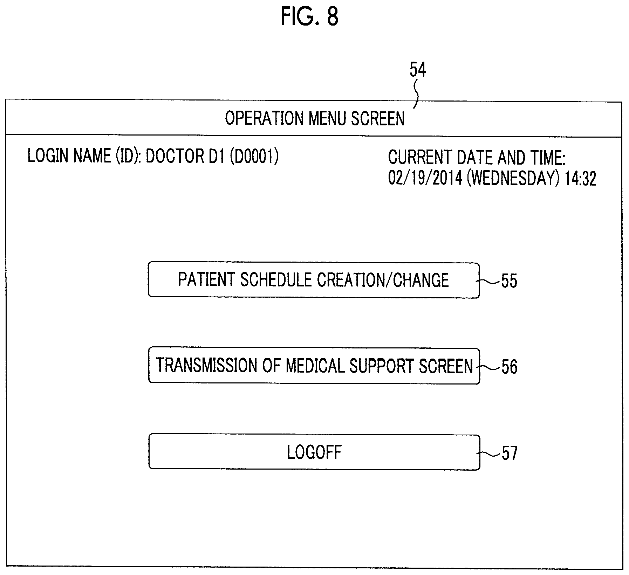

The receiving unit 47 transmits various kinds of operation screens including an operation menu screen 54 illustrated in Fig. 8 to the client terminals 13a to 13c which have been allowed to access the team medical support device by the authentication unit 46 and receives operations which have input through the operation screens of the client terminals 13a to 13c as operation information. The operation menu screen 54 is provided with a plurality of operation menu buttons 55 to 57. In a case in which the client terminals 13a to 13c are personal computers, for example, a mouse is operated to move a pointer on the screen and is clicked to select the operation menu buttons 55 to 57. In a case in which the client terminals 13a to 13c are portable terminals with touch panels, such as smart phones or tablet terminals, the operation menu buttons 55 to 57 are directly selected on the touch panel. In case one of the operation menu buttons 55 to 57 of the client terminals 13a to 13c is operated, operation information indicating which of the operation menu buttons is operated is transmitted from the client terminals 13a to 13c to the receiving unit 47.

The "patient schedule creation/change" button 55 of the operation menu screen 54 is operated in case patient schedule information is newly created or in case the created patient schedule information is changed. In case the "patient schedule creation/change" button 55 is operated in the client terminals 13a to 13c, the receiving unit 47 transmits a patient schedule operation screen (not illustrated) to the client terminals 13a to 13c. The client terminals 13a to 13c can newly create patient schedule information and change the created patient schedule information through the patient schedule operation screen. The receiving unit 47 receives the patient schedule information which has been newly created by the client terminals 13a to 13c and the patient schedule information which has been changed by the client terminals 13a to 13c and inputs the information to the patient schedule information creation/update unit 48. The patient schedule information creation/update unit 48 gives a patient schedule ID to the newly created patient schedule information and stores the newly created patient schedule information in the patient schedule information storage unit 16. In addition, for the changed patient schedule information, the patient schedule information creation/update unit 48 rewrites a changed portion in the patient schedule information storage unit 16.

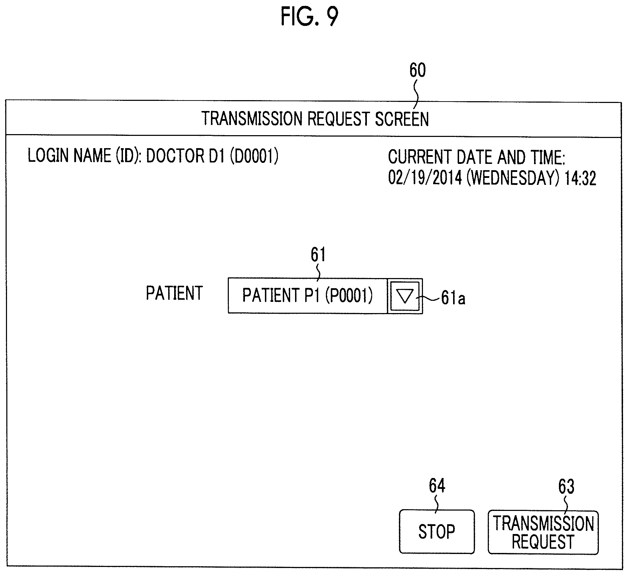

In Fig. 8, the "medical support screen transmission" button 56 of the operation menu screen 54 is operated in case there is a request to transmit the medical support screen 20 (see Fig. 1) . In case the "medical support screen transmission" button 56 is operated in the client terminals 13a to 13c, the receiving unit 47 transmits a transmission request screen 60 illustrated in Fig. 9 to the client terminals 13a to 13c. The transmission request screen 60 includes a patient designation field 61 for designating a patient corresponding to the medical support screen 20. In case a list display button 61a which is provided at the right end of the patient designation field 61 is operated, a list of the patients stored in the patient information storage unit 24 is displayed such that a patient can be selected and designated from the displayed list.

A transmission request button 63 for instructing the transmission of a transmission request and a stop button 64 for stopping the transmission request are provided in a lower right portion of the transmission request screen 60. In case the transmission request button 63 is operated, a transmission request is transmitted as the operation information of the client terminals 13a to 13c to the team medical support device 11. The transmission request includes the patient ID of the patient designated in the patient designation field 61 as patient designation information for designating a patient corresponding to the medical support screen 20. In case the stop button 64 is operated, the display screen of the client terminals 13a to 13c returns from the transmission request screen 60 to the operation menu screen 54.

In Fig. 7, in a case in which the receiving unit 47 receives a transmission request from the client terminals 13a to 13c, the screen creation unit 49 reads the patient schedule information which corresponds to the patient ID designated by the transmission request from the patient schedule information storage unit 16 and creates the medical support screen 20 on the basis of the read patient schedule information.

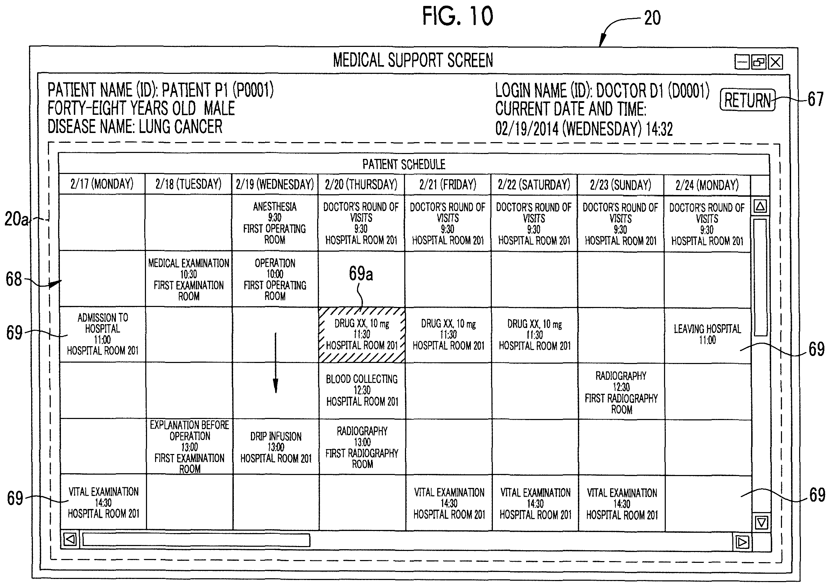

As illustrated in Fig. 10, the patient schedule information display region 20a is provided on the medical support screen 20 which is created first in response to the transmission request so as to occupy substantially the entire region of the medical support screen 20, but the communication message display region 20b (see Fig. 1) is not provided. In a case in which event information in the patient schedule information is selected on the medical support screen 20, the communication message display region 20b is provided in the medical support screen 20 in order to summarize and display the communication messages related to the selected event information.

A login name which is the name of a medical staff member who requests the creation of the medical support screen 20, a login ID, and the current date and time are displayed in an upper right portion of the medical support screen 20. The patient name, patient ID, age, sex, and disease name of a patient corresponding to the medical support screen 20 are displayed in an upper left portion of the screen. In addition, a return button 67 which is operated in a case in which the medical support screen 20 returns to the operation menu screen 54 is provided in the upper right end of the medical support screen 20. The patient schedule information display region 20a is provided substantially the entire region of the medical support screen 20. A patient schedule table 68 in which the patient schedule information is arranged in the form of a table is provided in the patient schedule information display region 20a. The date and time is allocated in chronological order along the horizontal axis and the vertical axis of the patient schedule table 68 and the patient schedule table 68 comprises a plurality of cells 69 which correspond to the date and time and are arranged in a matrix. The event information of the patient schedule information is displayed in each cell 69.

In Fig. 10, the medical support screen 20 is a medical support screen of the patient P1 and the patient schedule information (patient schedule ID: PS0001) of the patient P1 illustrated in Fig. 2 is displayed in the patient schedule table 68. A plurality of event information items, such as admission to a hospital at 11:00 on February 17, an operation until 10:00 on February 19, and leaving the hospital at 11:00 on February 24, are displayed in each cell 69 of the patient schedule table 68.

In Fig. 7, the transmission unit 51 transmits the medical support screen 20 which has been created by the screen creation unit 49 on the basis of the transmission request to the client terminals 13a to 13c which have transmitted the transmission request.

In a case in which the communication messages related to the event information are collectively checked, a cell 69 which is displayed in the patient schedule table 68 of the medical support screen 20 is selected in the client terminals 13a to 13c. In case event information is selected by the selection of the cell 69, event selection information indicating that the event information has been selected is transmitted as the operation information of the client terminals 13a to 13c to the team medical support device 11. The event selection information includes the event ID of the selected event information. For example, the background color of the cell 69 in which the selected event information is displayed is changed such that the medical staff can recognize the selected state.

For example, in the patient schedule table 68, in a case in which a cell 69a "February 20, 11:30" is selected, the background color of the cell 69a is changed and event selection information including an event ID "EV10009" of the event information displayed in the cell 69a is transmitted from the client terminals 13a to 13c to the team medical support device 11.

In Fig. 7, in a case in which the receiving unit 47 receives the event selection information, the screen creation unit 49 reads a communication message related to the selected event information, specifically, a communication message in which the same event ID as that included in the event selection information is recorded, from the communication message storage unit 18, and re-creates the medical support screen 20 having the communication message display region 20b.

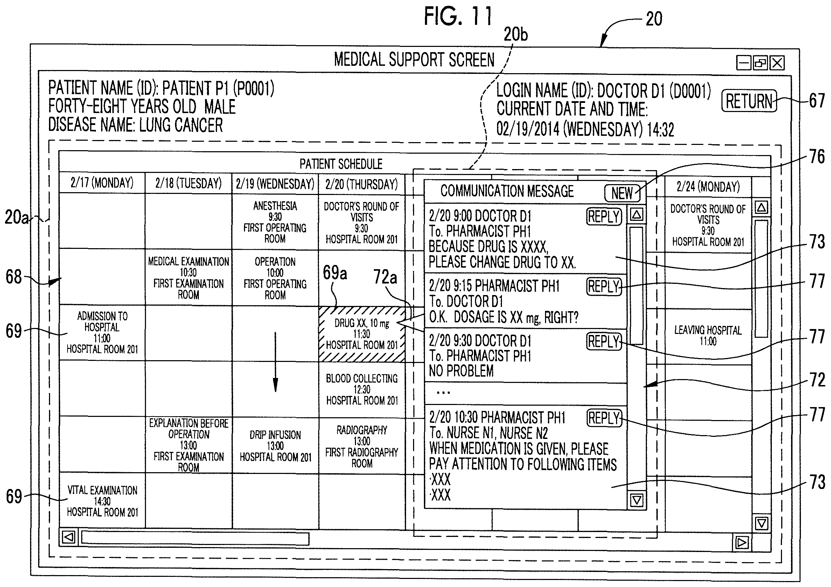

As illustrated in Fig. 11, the communication message display region 20b is provided on the medical support screen 20 which has been re-created by the selection of the event information so as to be superimposed on the patient schedule information display region 20a. A message list 72 in which communication messages related to the selected event information are summarized and displayed is provided in the communication message display region 20b. The message list 72 comprises a plurality of message display fields 73. For example, communication messages are displayed in each message display field 73 in the order of the transmission date and time. In addition, the message list 72 is connected to the cell 69 by a leader line 72a such that the relation of the message list 72 to the selected event information can be easily recognized.

For example, in a case in which event information displayed in a cell 69a is selected in the patient schedule table 68, communication messages which are related to the event information displayed in the cell 69a is summarized and displayed in the message list 72 in the communication message display region 20b. The event information with the event ID "EV10009" illustrated in Fig. 2 is displayed in the cell 69a. Therefore, the transmission date and time, transmission source, transmission destination, and text of communication messages "MS0003" to "MS0006" related to an event ID "EV10009" among a plurality of communication messages illustrated in Fig. 3 are displayed in a plurality of message display fields 73 of the message list 72 in the order of the transmission date and time.

In Fig. 7, the transmission unit 51 re-transmits the medical support screen 20 which has been re-created by the screen creation unit 49 with the selection of the cell 69 to the client terminals 13a to 13c which have selected the cell 69.

A new button 76 and a reply button 77 are provided in the message list 72 . The new button 76 is arranged in a title portion in which a name is displayed in the message list 72 and is operated in a case in which communication messages related to the selected event information are newly transmitted. The reply button 77 is arranged in each of the message display fields 73 of the message list 72 and is operated in a case in which a reply to the communication message displayed in the message display field 73 is made.

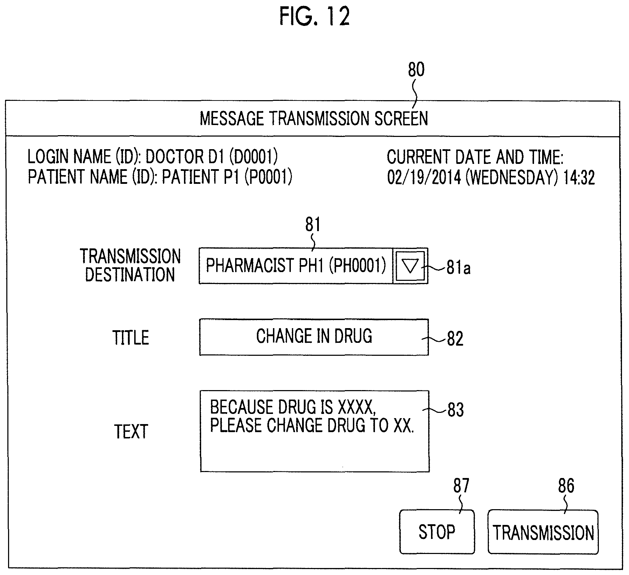

In Fig. 7, in a case in which the receiving unit 47 receives operation information indicating the operation of the new button 76 or the reply button 77, a message transmission screen is transmitted to the client terminals 13a to 13c which have transmitted the operation information. A message transmission screen 80 illustrated in Fig. 12 is a screen which is transmitted in a case in which the new button 76 is operated and comprises a transmission destination designation field 81 for designating the transmission destination of a communication message, a title input field 82 for inputting the title of the communication message, and a text input field 83 for inputting text of the communication message. In case a list display button 81a which is provided at the right end of the transmission destination designation field 81 is operated, a list of the medical staff members stored in the staff information storage unit 25 is displayed. A transmission destination can be selected and designated from the displayed list. In addition, a plurality of medical staff members may be selected from the transmission destination designation field 81.

A transmission button 86 for instructing the transmission of a communication message and a stop button 87 for stopping the transmission of a communication message are provided in a lower right portion of the message transmission screen 80. In case the transmission button 86 is operated, a communication message is transmitted as the operation information of the client terminals 13a to 13c to the team medical support device 11. The communication message includes a transmission source ID, a transmission date and time, a target patient ID, and an event ID, in addition to each information item which is input through the message transmission screen 80. A login ID which is used to log in the team medical support device 11, a patient ID designated by a transmission request, and the event ID of selected event information are used as the transmission source ID, the patient ID, and the event ID, respectively.

In Fig. 12, a communication message transmission screen which is stored in a record corresponding to the message ID "MS0003" illustrated in Fig. 3 is illustrated on the message transmission screen 80. "PH001" is input to the transmission destination designation field 81, "change in drug" is input to the title input field 82, and "Because drug is XXXX, please change the drug to XX." is input to the text input field 83.

In a case in which the reply button 77 is operated in the message list 72 on the medical support screen 20, a message transmission screen (not illustrated) for reply is transmitted to the client terminals 13a to 13c. The message transmission screen for reply differs from the message transmission screen 80 for new transmission illustrated in Fig. 12 in that the transmission destination designation field 81 is omitted. Since the transmission destination of a communication message to be replied is predetermined, the transmission destination designation field 81 is omitted from the message transmission screen for reply. In case a title and text are input through the message transmission screen for reply and the transmission button is operated, a communication message including a transmission destination ID, a transmission source ID, a transmission date and time, a target patient ID, and an event ID in addition to each information item input through the message transmission screen is transmitted from the client terminals 13a to 13c to the team medical support device 11.

In Fig. 7, the message relay unit 50 gives a message ID to the communication message received by the receiving unit 47 and stores the communication message in the communication message storage unit 18.

In a case in which there is no communication message related to the selected event information, the message list 72 is not displayed in the communication message display region 20b. Therefore, in a case in which there is no communication message related to the selected event information, a small screen (not illustrated) in which the new button 76 for newly transmitting a communication message is provided is displayed in the communication message display region 20b and the message transmission screen 80 can be called by the new button 76.

In Fig. 8, the "log-off" button 57 of the operation menu screen 54 is operated in case a user logs off the team medical support device 11. In case the "log-off" button 57 is operated in the client terminals 13a to 13c, the authentication unit 46 illustrated in Fig. 7 cancels the authenticated state of the client terminals 13a to 13c.

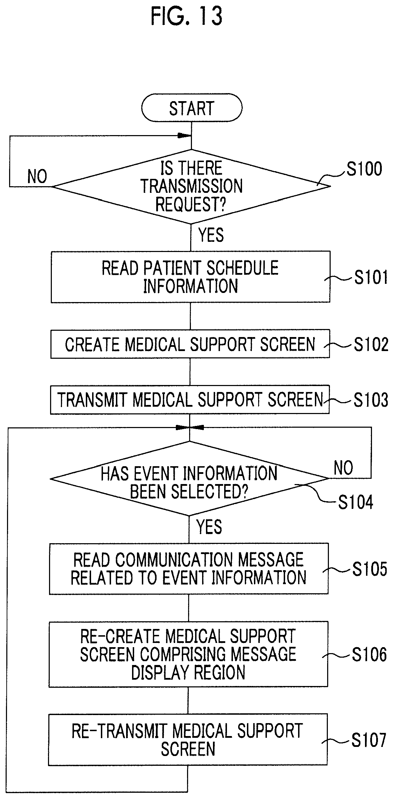

Next, the operation of the above-mentioned structure will be described with reference to a flowchart illustrated in Fig. 13. The team medical support device 11 waits for a request to transmit the medical support screen 20 from the client terminals 13a to 13c (S100). In case a transmission request from the client terminals 13a to 13c is received (YES in S100) , the screen creation unit 49 reads the patient schedule information of a patient designated by the transmission request from the patient schedule information storage unit 16 (S101) and creates the medical support screen 20 on the basis of the read patient schedule information (S102). The transmission unit 51 transmits the created medical support screen 20 to the client terminals 13a to 13c which have transmitted the transmission request (S103).

In case of receiving the transmitted medical support screen 20, the client terminals 13a to 13c display the medical support screen 20 on the displays. The patient schedule table 68 in which the patient schedule information read from the patient schedule information storage unit 16 is arranged in the form of a table is displayed in the patient schedule information display region 20a of the medical support screen 20. The date and time is allocated in chronological order along the horizontal axis and the vertical axis of the patient schedule table 68 and the event information is displayed in a plurality of cells 69 which are arranged in a matrix in correspondence with the date and time.

The team medical support device 11 waits for the selection of event information from the patient schedule table 68 in the client terminals 13a to 13c (S104). In case of receiving event selection information from the client terminals 13a to 13c (YES in S104), the screen creation unit 49 reads a communication message related to the event ID which is designated by the event selection information from the communication message storage unit 18 (S105) and re-creates the medical support screen 20 comprising the communication message display region 20b on the basis of the read communication message (S106). The transmission unit 51 re-transmits the re-created medical support screen 20 to the client terminals 13a to 13c which have selected the event information (S107).

The message list 72 in which the communication messages related to the selected event information are summarized is displayed in the communication message display region 20b of the medical support screen 20. The message list 72 is connected to the cell 69 by the leader line 72a such that the relation of the message list 72 to the selected event information can be easily recognized.

As described above, the team medical support device 11 according to the invention creates the medical support screen 20 comprising the patient schedule information display region 20a in which the patient schedule information of a patient designated by a transmission request from the client terminals 13a to 13c used by the medical staff is displayed and the communication message display region 20b in which the communication messages related to event information selected from the patient schedule information are summarized and displayed, in response to the transmission request, and transmits the medical support screen 20 to the client terminals 13a to 13c which have transmitted the transmission request. Only the patient schedule information of one patient and the communication message related to the selected event information are displayed on the medical support screen 20. Therefore, content is briefly displayed and it is easy to ascertain the relation between the patient schedule information and the communication message. In addition, it is possible to collectively check the communication messages related to a medical procedure that is scheduled to be performed for the patient indicated by event information. Therefore, it is possible to rapidly and accurately ascertain the content of the communication message and the medical staff members smoothly exchange and share information in a team medical treatment.

In the above-described embodiment, the case in which one event information item is selected from the patient schedule table 68 has been described. However, in a case in which a plurality of event information items are selected, a plurality of message lists 72 in which communication messages related to each of the selected event information items are summarized may be displayed. In addition, the text of the communication message is displayed in the message display field 73 of the message list 72. However, a portion of the text or a title may be displayed. The communication messages are displayed in the order of the transmission date and time. However, the display order may be appropriately changed. For example, the communication messages are displayed in the order of the degree of importance, the degree of urgency, the medical staff, or unconfirmed messages. The message list 72 is displayed so as to be superimposed on the patient schedule table 68. However, the message list 72 may be displayed in parallel to the patient schedule table 68.

Next, second to fifth embodiments of the invention will be described. The same structures as those in the first embodiment are denoted by the same reference numerals and the detailed description thereof will not be repeated.

### [Second Embodiment]

In the first embodiment, the communication messages which are related to event information selected from the patient schedule information displayed in the patient schedule information display region 20a are summarized and displayed in the communication message display region 20b of the medical support screen 20. However, in some cases, the medical staff wants to collectively check communication messages related to the entire plan for examining a patient, in addition to the communication messages related to a limited medical procedure, such as event information, in order to check or review the entire plan for examining the patient in a team medical treatment. In this embodiment, communication messages related to a patient are summarized and displayed in a communication message display region of a medical support screen. Therefore, medical staff members smoothly exchange and share information in the team medical treatment.

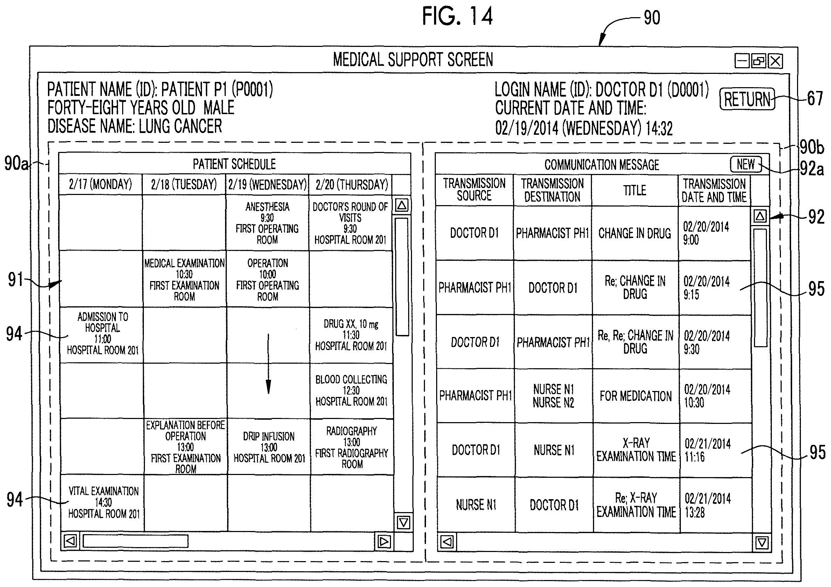

On a medical support screen 90 illustrated in Fig. 14, a patient schedule information display region 90a and a communication message display region 90b are displayed in parallel in the horizontal direction. Among the patient schedule information items of a plurality of patients stored in the patient schedule information storage unit 16, patient schedule information of a patient designated by a transmission request from the client terminals 13a to 13c is displayed in a patient schedule table 91 in the patient schedule information display region 90a. Among a plurality of communication messages stored in the communication message storage unit 18, communication messages which are related to the patient designated by the transmission request are summarized and displayed in a message list 92 in the communication message display region 90b.

The date and time is allocated in chronological order along the horizontal axis and the vertical axis of the patient schedule table 91 and the patient schedule table 91 comprises a plurality of cells 94 which correspond to the date and time and are arranged in a matrix. The event information of the patient schedule information is displayed in each cell 94. The transmission source, transmission destination, title, and transmission date and time of a communication message are displayed in each message display field 95 of the message list 92 in reverse chronological order of the transmission date and time from the upper side. In addition, a portion of the text of the communication message may be displayed in the message display field 95, instead of the title or together with the title.

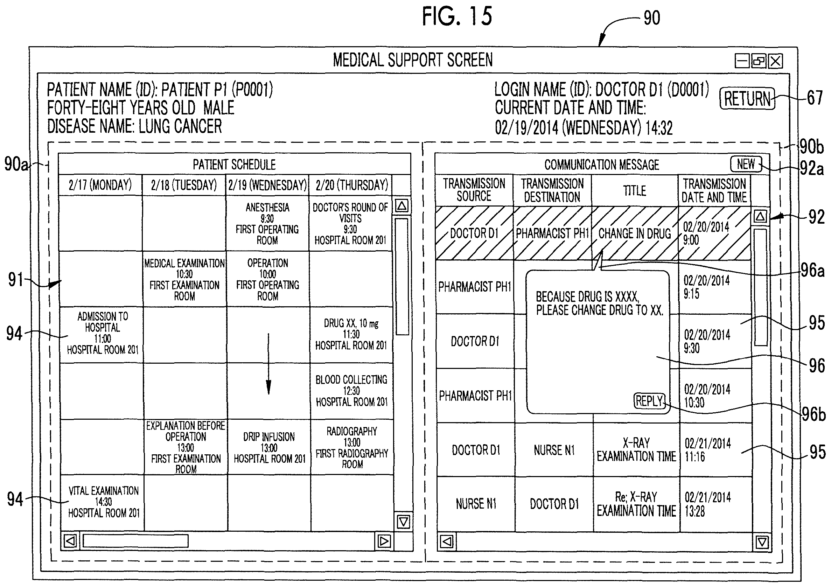

In case a communication message is selected from the message list 92, a text display field 96 in which the text of the selected communication message is displayed is displayed so as to be superimposed on the message list 92, as illustrated in Fig. 15. Since the text display field 96 is connected to the communication message by a leader line 96a, the medical staff can easily understand which message the text belongs to.

A new button 92a which is operated in case a communication message related to a patient is newly transmitted is provided in a title portion of the message list 92. In case the new button 92a is operated, the same message display screen (not illustrated) as the message transmission screen 80 for transmitting a new message illustrated in Fig. 12 is displayed so as to be superimposed on the medical support screen 90 and the communication message input to the message display screen can be transmitted. In addition, a reply button 96b which is operated in case a reply to the displayed communication message is made is provided in the text display field 96. In case the reply button 96b is operated, the same message display screen (not illustrated) as the message transmission screen for reply which has been described in the first embodiment is displayed so as to be superimposed on the medical support screen 90 and it is possible to reply to the communication message input to the message display screen.

As described above, according to the medical support screen 90 of this embodiment, the communication messages related to the patient designated by the transmission request are summarized and displayed in the message list 92 in the communication message display region 90b that is displayed in parallel to the patient schedule information display region 90a. Therefore, it is possible to collectively check the communication messages related to the entire plan for examining the patient. As a result, it is possible to easily check or review the entire plan for examining the patient and medical staff members smoothly exchange and share information.

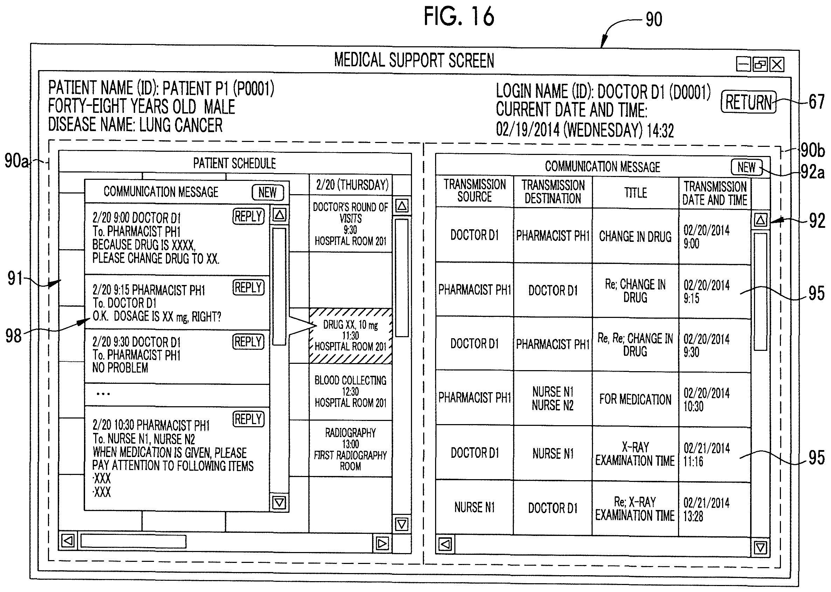

In this embodiment, the patient schedule information display region 90a and the communication message display region 90b are displayed in parallel. However, this display form may be combined with the display form of the communication message according to the first embodiment. As illustrated in Fig. 16, in a case in which event information is selected in the patient schedule information display region 90a, a message list 98 in which communication messages related to the selected event information are summarized may be displayed so as to be superimposed on the patient schedule table 91. In this case, it is possible to collectively check communication messages related to event information on the same screen, in addition to the communication messages related to the patient, if necessary.

### [Third Embodiment]

In the first embodiment, all of the communication messages related to event information are displayed in the communication message display region 20b of the medical support screen 20. However, the job type of the medical staff who participates in a team medical treatment is not limited to a doctor, a nurse, an X-ray technician, and a pharmacist, but is various. Therefore, it is necessary to protect detailed information related to a medical examination in terms of the protection of personal information or medical ethics, according to the job type of the medical staff. In this embodiment, the communication messages which are displayed in the communication message display region 20b of the medical support screen 20 are protected according to the access authority of the medical staff such that the medical staff members smoothly exchange and share information in the team medical treatment.

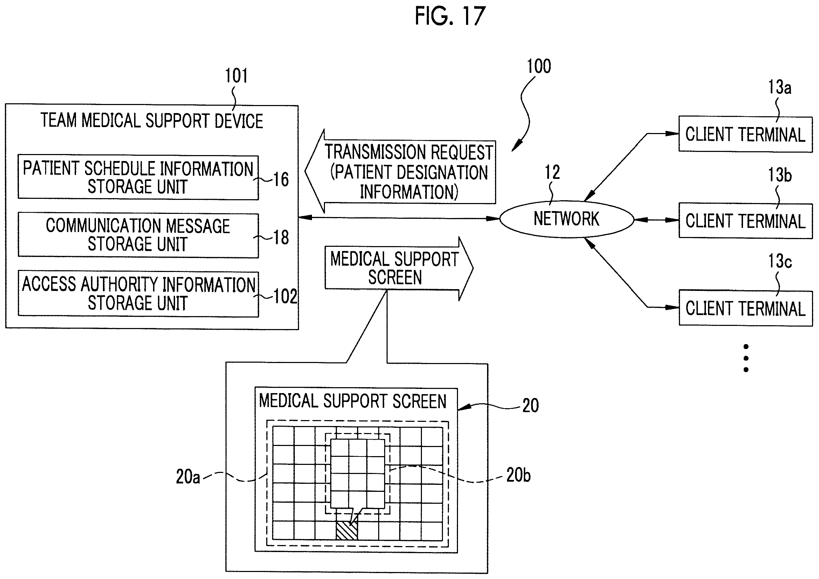

A team medical support device 101 of a team medical support system 100 illustrated in Fig. 17 differs from the team medical support device 11 according to the first embodiment in that it further includes an access authority information storage unit 102 that stores the access authority information of the medical staff for the communication message display region 20b. For example, the type of communication message which is not allowed to be accessed is registered for each medical staff member and each job type of the medical staff in the access authority information storage unit 102. Examples of the type of communication message in which the access authority information is set include a communication message which is transmitted between medical staff members of a specify job type, such as a communication message which is transmitted from a doctor to another doctor, and a communication message which is transmitted between specific medical staff members, such as a communication message which is transmitted from a doctor D1 to a nurse N1. In addition, access authority may be set according to the degree of urgency of the communication message.

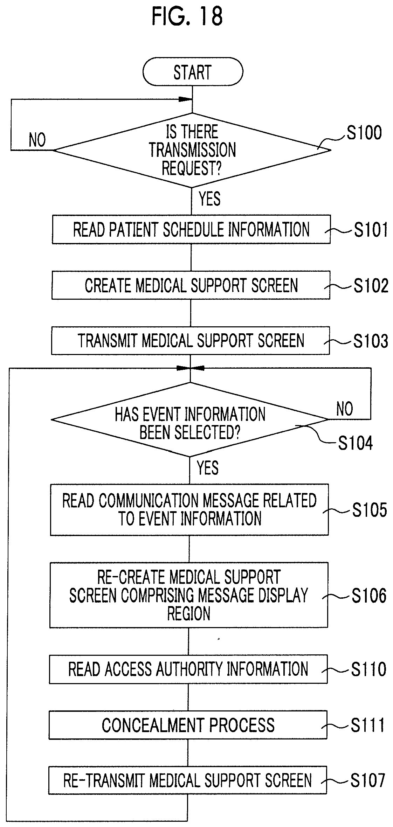

As illustrated in Fig. 18, the team medical support device 101 waits for the selection of event information in the client terminals 13a to 13c (S104). In case event selection information is received from the client terminals 13a to 13c (YES in S104), the screen creation unit 49 reads a communication message related to an event ID designated by the event selection information from the communication message storage unit 18 (S105) and re-creates the medical support screen 20 comprising the communication message display region 20b on the basis of the read communication message (S106).

The screen creation unit 49 reads the access authority information of a medical staff member who has transmitted the transmission request from the access authority information storage unit 102 (S110). In a case in which there is a communication message which is not allowed to be accessed by the access authority information among the communication messages displayed in the communication message display region 20b, the screen creation unit 49 performs a concealment process of concealing the communication message which is not allowed to be accessed from the communication message display region 20b (S111). After the communication message concealment process is completed, the transmission unit 51 transmits the medical support screen 20 to the client terminals 13a to 13c which have transmitted the transmission request (S107).

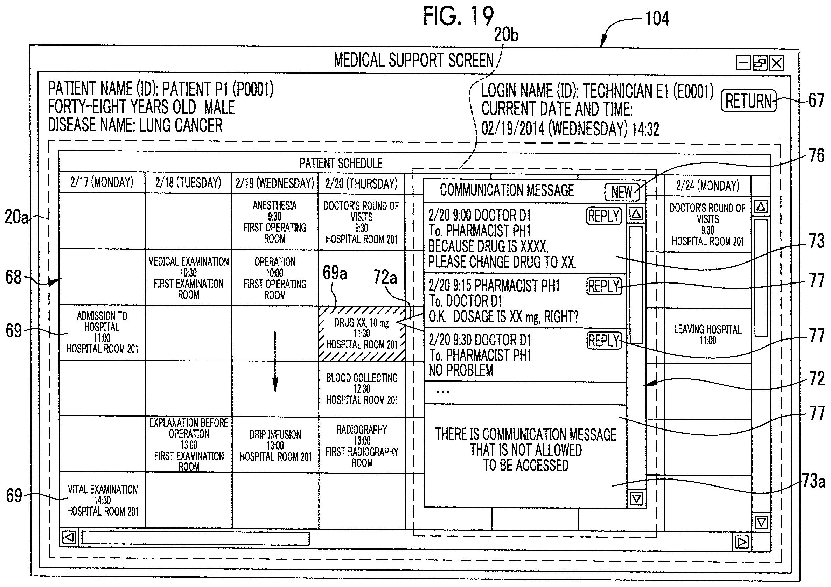

Fig. 19 illustrates a medical support screen 104 of a patient P1 of which the transmission has been requested by a technician E1 who is an X-ray technician. In a message list 72 in a communication message display region 20b, a concealment process which prevents a communication message from being displayed is performed for a message display field 73a which is not allowed to be accessed by the technician E1. In addition, in the concealment process, a message "there is a communication message that is not allowed to be accessed" is displayed in the message display field 73a subjected to the concealment process.

As described above, in this embodiment, in a case in which the communication messages displayed in the communication message display region include a communication message which is not allowed to be accessed by a medical staff member to which the medical support screen is transmitted, the team medical support device 101 performs the concealment process of concealing the communication message which is not allowed to be accessed. Therefore, it is possible to protect the personal information of patients or information to be protected in terms of medical ethics from the medical staff who does not have access authority. In addition, since information indicating that there is a communication message which is not allowed to be accessed is displayed in the display field of the communication message subjected to the concealment process, a medical staff member can recognize that there is a communication message which is not allowed to be accessed, participate in a team medical treatment, and smoothly cooperate with other medical staff members. Therefore, the medical staff members smoothly exchange and share information in the team medical treatment.

### [Fourth Embodiment]

In the first embodiment, the team medical support device 11 receives the communication messages transmitted from the client terminals 13a to 13c and stores the communication messages in the communication message storage unit 18. In a case in which a medical support screen transmission request is received from the client terminals 13a to 13c, the team medical support device 11 displays the communication messages on the medical support screen 20 and transmits the medical support screen 20 to the client terminals 13a to 13c. Therefore, in case the transmitted medical support screen is not received, a medical staff member is not capable of checking new communication messages transmitted from other medical staff members. As a result, the medical staff members do not smoothly exchange and share information. This embodiment provides a structure which enables medical staff members to rapidly recognize the presence of a new communication message and to smoothly exchange and share information in a team medical treatment.

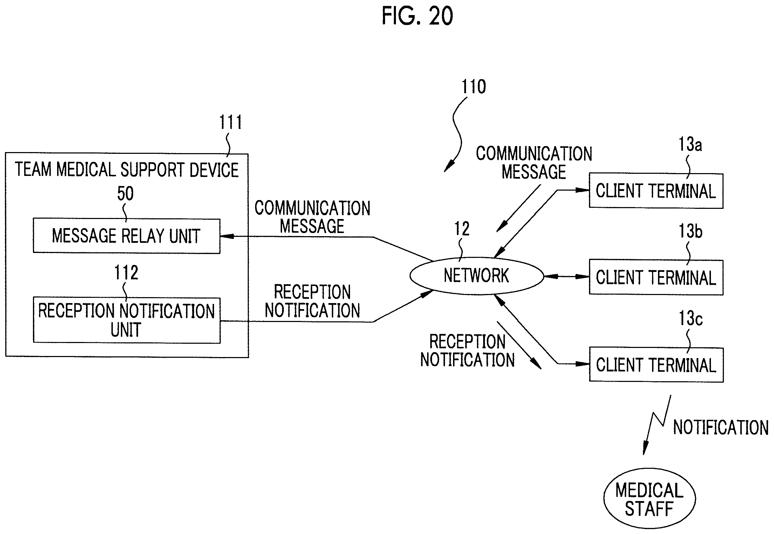

A team medical support device 111 of a team medical support system 110 illustrated in Fig. 20 differs from the team medical support device 11 according to the first embodiment in that it further includes a reception notification unit 112. The CPU 36 implements the functions of the reception notification unit 112 in cooperation with, for example, the memory 37, similarly to the message relay unit 50.

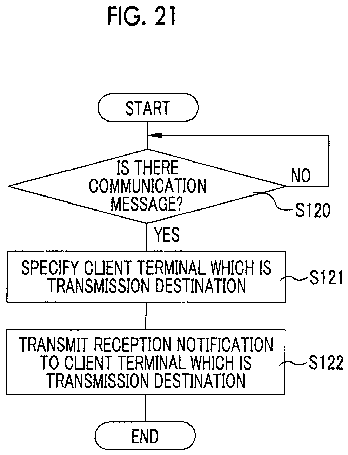

As illustrated in Fig. 21, the message relay unit 50 of the team medical support device 111 waits for the transmission of a communication message from the client terminals 13a to 13c (S120). For example, in case the message relay unit 50 receives a communication message from the client terminal 13a (YES in S120), the reception notification unit 112 specifies a client terminal which is the transmission destination of the received communication message (S121). The client terminal which is the transmission destination is specified by searching for a medical staff member having the same staff ID as the transmission destination ID of the received communication message from the staff information storage unit 25 and acquiring the terminal ID of the client terminal used by the searched medical staff member from the staff information storage unit 25. For example, in a case in which the client terminal which is the transmission destination is the client terminal 13c, the reception notification unit 112 transmits a notification indicating the reception of the communication message to the client terminal 13c (S122).

The client terminal 13c notifies the medical staff member of the reception of the communication message, using, for example, a sound, light, vibration, or the display of a screen. In case of receiving the communication message reception notification, the medical staff member can request the team medical support device 111 to transmit a medical support screen, check the medical support screen, and check the content of the communication message.

As described above, in this embodiment, in a case in which a communication message is received from a client terminal, the team medical support device 111 transmits a reception notification to the client terminal which is the transmission destination of the received communication message. Therefore, a medical staff member who has received the transmitted communication message can rapidly recognize the presence of the communication message, without checking the medical support screen. As a result, the medical staff members smoothly exchange and share information in a team medical treatment. In case the reception notification is transmitted, the degree of urgency of the communication message may also be transmitted and the aspect of the notification from the client terminals 13a to 13c to the medical staff member may vary depending on the degree of urgency. In this case, the medical staff members can check the reception of communication messages with a high degree of urgency.

In each of the above-described embodiments, in case a communication message is transmitted, the staff ID of a medical staff member who is a transmission destination is designated. However, for example, the patient ID may be designated and a communication message may be transmitted to all of the medical staff members related to a medical examination for the patient.

In each of the above-described embodiments, the team medical support device is installed in the medical institutions. However, the team medical support device may be installed in, for example, data centers or local medical centers. In this case, the team medical support device is connected to the client terminals 13a to 13c in the medical institution through, for example, the Internet, a dedicated communication line, or a virtual private network (VPN). In each of the above-described embodiments, for example, the patient schedule information storage unit 16, the communication message storage unit 18, the patient information storage unit 24, and the staff information storage unit 25 are table-type databases. However, databases in which each patient, each medical staff member, and each communication message are managed as files may be used.

## Claims

1. A team medical support device comprising:
a patient schedule information storage unit that stores patient schedule information of a plurality of patients in which event information indicating a medical procedure that is performed for the patient is registered;
a communication message storage unit that stores a plurality of communication messages which are exchanged between client terminals used by a plurality of medical staff members;
a receiving unit that receives a transmission request including patient designation information for designating one patient among the plurality of patients;
a screen creation unit that creates a medical support screen comprising a patient schedule information display region in which the patient schedule information of the patient designated by the patient designation information among the patient schedule information items of the plurality of patients is displayed and a communication message display region in which a communication message related to the patient designated by the patient designation information or a communication message related to at least one said event information item of the patient designated by the patient designation information among the plurality of communication messages is summarized and displayed; and
a transmission unit that transmits the medical support screen to the client terminal which has transmitted the transmission request.

2. The team medical support device according to claim 1,
wherein, in a case in which the screen creation unit creates the medical support screen including the communication message display region in which the communication message related to at least one event information item is summarized, the screen creation unit creates a medical support screen on which only the patient schedule information display region is displayed and transmits the medical support screen, and
in a case in which at least one event information item is selected from the medical support screen transmitted to the client terminal, the screen creation unit re-creates the medical support screen comprising the communication message display region in which a communication message related to the selected event information item is summarized and displayed and transmits the medical support screen.

3. The team medical support device according to claim 1,
wherein the communication message display region is displayed so as to be superimposed on the patient schedule information display region and is connected to the event information selected in the patient schedule information display region by a leader line.

4. The team medical support device according to claim 1,
wherein the communication message display region is displayed in parallel to the patient schedule information display region.

5. The team medical support device according to claim 1,
wherein a transmission date and time of the communication message is displayed in the communication message display region.

6. The team medical support device according to claim 1,
wherein a transmission source and a transmission destination of the communication message are displayed in the communication message display region.

7. The team medical support device according to claim 1,
wherein a title of the communication message or at least a portion of text of the communication message is displayed in the communication message display region.

8. The team medical support device according to claim 1,
wherein the communication messages are displayed in the communication message display region in an order of transmission date and time.

9. The team medical support device according to claim 1, further comprising:
an access authority information storage unit that stores access authority information of the medical staff member for the communication message display region,
wherein, in case of creating the medical support screen, the screen creation unit checks the access authority information of the medical staff member who is a request source of the transmission request, and
in a case in which the communication messages displayed in the communication message display region include a communication message that is not allowed to be accessed by the medical staff member, the screen creation unit performs a concealment process of concealing the communication message that is not allowed to be accessed.

10. The team medical support device according to claim 9,
wherein the concealment process includes a process of displaying information indicating that the communication message which is not allowed to be accessed by the medical staff member is present.

11. The team medical support device according to claim 1, further comprising:
a message relay unit that receives the communication message transmitted from the client terminal and relays the communication message; and
a reception notification unit that transmits a notification indicating the reception of the communication message to the client terminal used by the medical staff member who is a transmission destination of the received communication message.

12. A method for controlling a team medical support device, comprising:
a receiving step of receiving a transmission request including patient designation information for designating one patient among a plurality of patients;
an information reading step of accessing a patient schedule information storage unit that stores patient schedule information of a plurality of patients in which event information indicating a medical procedure performed for the patient is registered and a communication message storage unit that stores a plurality of communication messages which are exchanged between client terminals used by a plurality of medical staff members and reading the patient schedule information of the patient designated by the transmission request and the communication message;
a screen creation step of creating a medical support screen comprising a patient schedule information display region in which the patient schedule information read in the information reading step is displayed and a communication message display region in which a communication message related to the patient designated by the patient designation information or a communication message related to one said event information item of the patient designated by the patient designation information among the communication messages read in the reading step is summarized and displayed; and
a transmission step of transmitting the medical support screen to the client terminal which has transmitted the transmission request.

13. A team medical support program that causes a computer to perform:
a receiving step of receiving a transmission request including patient designation information for designating one patient among a plurality of patients;
an information reading step of accessing a patient schedule information storage unit that stores patient schedule information of a plurality of patients in which event information indicating a medical procedure performed for the patient is registered and a communication message storage unit that stores a plurality of communication messages which are exchanged between client terminals used by a plurality of medical staff members and reading the patient schedule information of the patient designated by the transmission request and the communication message;
a screen creation step of creating a medical support screen comprising a patient schedule information display region in which the patient schedule information read in the information reading step is displayed and a communication message display region in which a communication message related to the patient designated by the patient designation information or a communication message related to one said event information item of the patient designated by the patient designation information among the communication messages read in the reading step is summarized and displayed; and
a transmission step of transmitting the medical support screen to the client terminal which has transmitted the transmission request.

14. A team medical support system comprising client terminals that are used by a plurality of medical staff members, and a team medical support device that creates a medical support screen on the basis of a transmission request from the client terminals and transmits the medical support screen to the client terminals,
wherein the team medical support device comprises:
a patient schedule information storage unit that stores patient schedule information of a plurality of patients in which event information indicating a medical procedure that is performed for the patient is registered,
a communication message storage unit that stores a plurality of communication messages which are exchanged between the client terminals used by the plurality of medical staff members,
a receiving unit that receives a transmission request including patient designation information for designating one patient among the plurality of patients,
a screen creation unit that creates a medical support screen comprising a patient schedule information display region in which the patient schedule information of the patient designated by the patient designation information among the patient schedule information items of the plurality of patients is displayed and a communication message display region in which a communication message related to the patient designated by the patient designation information or a communication message related to at least one said event information item of the patient designated by the patient designation information among the plurality of communication messages is summarized and displayed, and
a transmission unit that transmits the medical support screen to the client terminal which has transmitted the transmission request.
